# EUROPEAN PATENT SPECIFICATION

(11) **EP 1 729 598 B1**
(45) Date of publication and mention of the grant of the patent: **10.09.2008**
(21) Application number: 05717277.7
(22) Date of filing: 14.03.2005
(51) Int. Cl.: A23L 1/307, A61K 31/25

(54) **COMPOSITION; USE OF A COMPOSITION AND A METHOD FOR PREVENTING FAT ABSORPTION**
ZUSAMMENSETZUNG, VERWENDUNG EINER ZUSAMMENSETZUNG UND VERFAHREN ZUR PRÄVENTION VON FETTAUFNAHME
COMPOSITION, UTILISATION D'UNE COMPOSITION ET PROCÉDÉ POUR PRÉVENIR L'ABSORPTION DE LIPIDES

(30) Priority: 15.03.2004 FI 20045076
(43) Date of publication of application: 13.12.2006
(73) Proprietor: Bioferme OY, 20760 Piispanristi (FI); Biolux OY, 00180 Helsinki (FI)
(72) Inventor: TUOMASJUKKA, Saska, FI-21555 Taatila (FI); KALLIO, Heikki, FI-21110 Naantali (FI)
(74) Representative: Nylund, Solveig Helena
(86) International application number: PCT/FI2005/000151
(87) International publication number: WO 2005/087025

(56) References cited:
- WO-A1-01/19340
- US-A1- 2003 027 786
- US-B1- 6 214 349
- US-B1- 6 703 369
- DATABASE WPI Week 199437, Derwent Publications Ltd., London, GB; Class B04, AN 1994-299697, XP002989013 & JP 6 227 996 A (NOPPON YAKUHIN KOGYO KK.) 16 August 1994

## Description

### BACKGROUND OF THE INVENTION

Fatness is becoming the world's severest health problem. WHO, the World Health Organization, estimates that the number of people suffering from obesity has exceeded 300 million.

In the USA, the authorities estimate that the proportion of fat people (weight index > 30) of the adult population is 27%. The proportion of overweight people (weight index 25 to 30) is about 33% of the adult population (NCCDPHP 2003). This is also a significant health problem in Finland: every fifth adult is overweight. The number of fat people in the industrialized countries has doubled in about twenty years, and no turn in the trend is in sight (WHO 2003). The underlying reason for fatness is decreased physical activity together with an increased or unchanged amount of energy in the nutrition.

In nutrition, fat contains the most energy, and with evolution, man increasingly values fat not only as a source of energy, but also because of its palatability. In almost all industrialized countries, the fat content in nutrition exceeds the recommendations. Continuous excessive intake of fat causes not only cosmetic harm associated with fatness, but also serious health problems and complications related to diseases, such as cardiovascular diseases, respiratory diseases and diabetes. Significant costs are also associated with said national diseases.

The most preferable way to prevent the drawbacks of excessive fat is increased exercise and adjustment of the energy obtained from food. Energy intake can be reduced for instance by observing a strict diet, by using low fat preparations or by preventing full-scale utilization of the energy in the food.

The aim at lowering the fat content in foodstuffs creates new challenges for the foodstuffs industry. The WHO and the food processing industry have started open discussions about the development of healthier future foodstuffs. Other expert organizations have also increased the pressure on the industry (e.g. IASO 2003). As a result, some foodstuffs companies have set an aim at improving the quality of fat and reducing its amount in the products.

Reduced-energy fat mixtures are described for instance in international patent publication WO 00/15043, and first fat compositions of this kind are entering the market. However, in Europe, the progress in slowed down by strict legislation involving authorization proceedings that take years.

A more efficient solution to the problem of excessive fat is to prevent fat from absorbing. As regards cholesterol, solutions based on this idea include for instance methods based on plant sterols and plant stanols, such as are described for instance in international patent publication WO 03/026672.

For instance American patent publication US 5,453,282 discloses chitosan-containing nutrients for preventing the absorption of the main component in fat, triacylglycerol (TAG), but, for the time being, the most effective prevention succeeds only by means of pharmacotherapy. The prescription medicine, orlistat (tetrahydrolipstatin), disclosed in European patent EP 129748, can be used to reduce the absorption of triacylglycerols by 30% (Zhi et al., 1994).

However, the problem in the use of orlistat is its relative ineffective-ness and the fact that pharmacotherapy is involved, requiring a visit to a physician.

WO-A-0 119 340 teaches a pharmaceutical composition comprising at least one lipase inhibitor, at least one surfactant and at least one dispersant. Preferred surfactants are poly(oxyethylene) sorbitan fatty acid esters, such as sorbitan mono-oleate.

To achieve the prevention of fat absorption by means of a functional food would be an extremely interesting solution to a serious and internationally recognized problem.

### BRIEF DESCRIPTION OF THE INVENTION

It has now unexpectedly been observed that a food-approved emulgator almost completely prevents fat from being absorbed from the digestive tract. Thus, the invention relates to the use of an emulgator for the preparation of a product intended for the prevention of fat absorption. The use of a food-approved emulgator in the preparation of such a product fulfils the security requirements set by the authorities, thus enabling the preparation of a functional food fulfilling the regulations. Such a preparation is the first functional food that shows almost complete prevention of fat absorption in humans.

The invention also relates to a food composition for preventing fat absorption, the emulgator being selected from the group consisting of sorbitan derivatives.

The invention also relates to a functional food characterized in that it comprises an effective amount of a food-approved sorbitan derivative.

The invention further relates to a pharmaceutical composition comprising an effective amount of an emulgator selected from the group consisting of sorbitan derivatives.

The invention also relates to the use of a sorbitan derivative for the preparation of a pharmaceutical product intended for the prevention of fat absorption.

The invention can be used for the prophylaxis, treatment or alleviation of obesity and for reducing the risks associated with fatness, by administering, to a human or animal suffering from obesity, a preparation containing an effective amount of a pharmaceutically acceptable emulgator for preventing fat absorption, and optionally other, pharmaceutically acceptable additives and carriers.

Preferred embodiments of the invention are described in the dependent claims.

### BRIEF DESCRIPTION OF THE FIGURES

In the following, preferred embodiments of the invention will be described in detail with reference to the accompanying drawings, in which
Figure 1 shows TAG concentrations measured from the plasma of subjects during 0 to 6 hours after intake of test product A;
Figure 2 shows TAG concentrations measured from the plasma of subjects during 0 to 6 hours after intake of test product B;
Figure 3 graphically shows the amount of fat absorbed by subjects as an iAUC value; and
Figure 4 graphically shows the amount of fat absorbed expressed as percentages per tested product.

### DETAILED DESCRIPTION OF THE INVENTION

The present invention is based on the surprising observation that a food-approved sorbitan derivative almost completely prevents fat absorption from the digestive tract. Said observation was made in a clinical trial whose aim was to find out how different treatments and formulations affect the absorption of valuable fats.

The invention thus relates to the use of a food-approved sorbitan derivative as an additive or ingredient in a food product, as a nutriment additive or a pharmaceutical preparation for achieving a health-enhancing effect, i.e. partial or complete prevention of the absorption of fat.

In this context, 'a food-approved sorbitan derivative refers to a compound, detergent, emulgator or stabilizing agent accepted as a food additive. In the use according to the present invention, preferable food-approved emulgators include sorbitan derivatives of Formula I 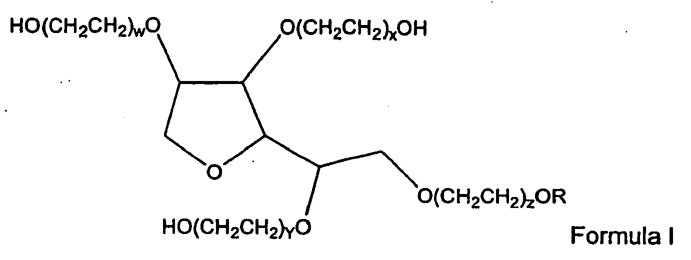 wherein
w + x + y + z = about 20, or w, x, y and z = 0,
and R is H, OOC-(CH₂)₁₀-CH₃, OOC-(CH₂)₁₄-CH₃, OOC-(CH₂)₇-(CH)₂-(CH₂)₇-CH₃ or 3XOOC-(CH₂)₁₆-CH₃.

Particularly preferable are sorbitan derivatives, such as sorbitan monostearate (E 491), sorbitan tristearate (E 492), sorbitan monolaurate (E 493), sorbitan monooleate (E 494) and sorbitan monopalmitate (E 495), or different polyoxyethylene sorbitan derivatives, such as -monolaurate (E 432), -monooleate (E 433), -monopalmitate (E 434), -monostearate (E 435) and -tristearate (E 436). A particularly preferable emulgator according to the invention is sorbitan monooleate (E 494).

The nutrient composition of the invention is the first functional food that has been proved to show an almost complete prevention of fat absorption in humans or animals.

In the present context, 'a functional food' refers to a nutrient/food product that has been shown to have, not only the conventional nutritional characteristics, but also a positive effect on one or more functions of the organism in a manner enhancing health or reducing the risk of disease.

The functional food according to the invention may be any food product, to which a sorbitan derivative according to the invention has been added, but preferable food products include beverages and other liquid food products, such as juices, milk beverages, gruel, soups and sauces. Examples of fat-containing food products or ingredients of food products, whereto the food-approved sorbitan derivative of the invention can be added, include fat emulsions for baking or other use, cream-imitation preparations, ice creams, emulsified sauces, such as hamburger sauces, salad sauces and dip sauces, thermally processed meat preparations, such as a cooked hamburger and different chocolates. The functional foods of the invention also include fatty food products, such as pastry, cookies, potato chips, hamburgers and chocolates, in whose preparation the above fatty emulsions are used. Other preferred forms of use are evident to a person skilled in the art, and the intention is not for the above examples to restrict other applications outside the scope of the present invention.

To the nutrient composition of the invention, such an amount of a food-approved sorbitan derivative is added that the desired effect is achieved. The effective amount may vary significantly depending on the composition of said food product and depending on the size of the dose of said food product to be ingested at a time. An effective amount of sorbitan derivative may also vary in accordance with the emulgator used, the food product and the amount of food product ingested. An effective amount of sorbitan derivative is 0.1 to 100 g/kg food product, preferably 1 to 25 g/kg, and most preferably 5 to 10 g/kg.

A preferable form of use according to the invention are separately consumed functional foods ingested in addition to normal nutrition, for example as a snack bar or an 'energy drink'.

Effective prevention of fat absorption can also be achieved by ingesting the sorbitan derivative of the invention as a separate supplement. The supplement of the invention may be for instance a capsule containing an effective amount of the sorbitan derivative of the invention. The capsule may be a soft or hard capsule, and the encapsulation material may be selected from the group consisting of gelatine, agar, starch, modified starch, modified starch power, carrageen, sugar, particularly sucrose, lactose or fructose.

If the nutrient composition of the invention is ingested as a supplement in addition to normal nutriment, a preferable amount of sorbitan derivative per dose to be ingested is more than 1 mg, preferably 1 to 10,000 mg, more preferably 100 to 5,000 mg, still more preferably 500 to 5,000 mg, and most preferably 2,000 mg. Such preferable amounts correspond to 0 to 65 mg/person's weight-kg, most preferably about 25 to 60 mg/kg. A skilled professional in the food product field, who is acquainted with nutrition, is easily able to determine the effective amount of emulgator required to achieve the desired effect on the basis of the above reference values and the experiments described in the examples.

The invention also relates to a pharmaceutical composition that can be a tablet, capsule, solution or emulsion depending on the sorbitan derivative used. The pharmaceutical composition of the invention can be prepared by using conventional methods. The pharmaceutical dosage form of the invention can be for instance a capsule containing an effective amount of the sorbitan derivative of the invention. The capsule may be a soft or hard capsule, the encapsulation material being selected from the group consisting of gelatine, starch, modified starch, modified starch powder, carrageen, sugar, particularly sucrose, lactose or fructose.

The sorbitan derivative containing pharmaceutical preparation of the invention can be prepared by using conventional methods (see for example Remington's Pharmaceutical Sciences, 16. volume, 1990). The therapeutically effective amount of sorbitan derivative per unit can be, according to the desired effect, 1 to 10,000 mg, preferably 100 to 5,000 mg, more preferably 500 to 5,000 mg, and most preferably 2,000 mg. In addition to the sorbitan derivative, the pharmaceutical preparation of the invention may also contain other additives and carriers generally used in the field, for instance pharmaceutically acceptable vegetable oils, such as paraffin oil, arrack oil, sesame oil, olive oil, soy oil and amygdale oil.

The present invention can be used for preventing partial absorption or complete absorption of fat from the digestive tract in an animal or human in need thereof, by administering an effective dose of the nutrient composition or pharmaceutical composition of the invention, containing a food-approved sorbitan derivative. The method achieves cosmetic or therapeutic weight control and thus prophylaxis of health hazards or diseases associated with excessive use of fat or a decrease in the risk of contracting a disease. The treatment can be administered either by ingestion of the functional food of the invention to replace part of normal nutrition, by ingestion of the functional food, for instance a beverage dose, in addition to normal fatty nutrition, or by ingestion of a separate supplement, for instance in capsule form, as a supplement to normal nutrition.

An effective single dose in connection with each meal, i.e. three times daily, is 500 mg/dose, which achieves the desired prevention of fat absorption.

### EXAMPLES

### EXAMPLE 1

### PREPARATION OF TEST PRODUCTS CONTAINING EMULSIFIED FAT

### Drink (Product A)

| Ingredient | % |
|---|---|
| 1. water | 72.81 |
| 2. concentrated juice mixture | 9.13 |
| 3. citric acid | 0.01 |
| 4. sugar | 7.03 |
| 5. blackcurrant aroma | 0.07 |
| 6. blackcurrant seed oil | 9.98 |
| 7. E494 | 0.98 |
| 8. vitamin A 2,800 IU / 100 g | |
| 9. vitamin E 20 IU / 100 g | |

### Preparation:

Ingredients 1 to 5 were mixed in accordance with a normal industrial preparation process. Ingredients 6, 7, 8 and 9 were added to the product under vigorous mixing.

### Oatmeal drink (Product B)

| Ingredient | g | % |
|---|---|---|
| 1. water | 70.00 | 67.6 |
| 2. oatmeal | 10.00 | 9.7 |
| 3. acidifier | 0.10 | 0.1 |
| 4. rapeseed oil | 0.45 | 0.4 |
| 5. berry preparation* | 12.00 | 11.6 |
| 6. blackcurrant seed oil | 9.97 | 9.6 |
| 7. E494 | 0.98 | 0.9 |
| 8. vitamin A 2,800 IU / 100 g | | |
| 9. vitamin E 20 IU / 100 g | | |

| | | |
|---|---|---|
| *) incl. fructose 6 %, mashed berries, pectin, aroma | | |

### Preparation:

Ingredients 1 to 3 are mixed and acidified in accordance with a normal industrial preparation process. Ingredients 4 and 5 are mixed into the acidified product. Ingredients 6, 7, 8 and 9 are added to the product under vigorous mixing.

Control products were prepared in accordance with the above instructions, but without ingredient 7.

### EXAMPLE 2

### ABSORPTION OF EMULSIFIED VEGETABLE OIL IN HUMANS

The purpose of the clinical trial was to study the absorption of some vegetable oils from different nutritive preparations. In the experiment, oil-containing test products were given to healthy human subjects. Blood sa mples were collected from the subjects and subjected to fat analyses, based on which the absorption of the oils was quantitatively assessed.

The ethical board of the Southwest Finland hospital district accepted the research plan of the experiment before the experiment was initiated. The subjects were randomized in respect of the products and the intake order thereof. In the morning of the day of the experiment, the subjects ingested the test product (product A) according to Example 1. The amount ingested was proportional to the weights of the subjects such that the amount of emulgator was about 65 mg/kilos of weight. The average single dose of the test product was thus 490 ml. The subjects in the control group ingested a corresponding oil-containing product (product A0), which did not contain emulgator. The next six hours the subjects spent under surveillance at the experimental laboratory, and altogether seven blood samples were taken from each them. The blood samples were collected before the test product was ingested and 30, 60, 180, 270 and 360 minutes thereafter. On the day following the experiment, the subjects were contacted in order to find out any acute side effects. Experimental day 2 was carried out after 2 to 4 weeks, the arrangements being similar except for the test product ingested.

The samples collected were either analyzed immediately or fractioned and frozen to wait for analyses to be performed later. Particularly, the composition of cylomicrons was studied, since the lipids contained therein directly reflect the fats and liposoluble compounds absorbed from a meal. The cylomicrons were separated from the plasma by ultracentrifugation and further fractioned into fatty acid and phospholipid fractions by means of micro silica columns. The fatty acids in the fractions were analyzed by gas chromatography. The results were subjected to statistical observation using the SPSS software.

The enzymatically measured triacyl glycerol content in the plasma suggested non-absorption of emulsified oil. The result was highly unexpected, since it seemed as if absorption was completely prevented.

The TAG analysis performed on the cylomicrons gave the same result. Consequently, test product A, which contained emulsified oil, was not at all absorbed.

Vitamin E and vitamin A as retinyl palmitate, which is soluble in the same way as fat, were added to the test products. It allows fat absorption, and, to some degree, the number of cylomicrons in the blood circulation, i.e. the discharge of fat from the blood circulation, to be monitored. No vitamin E and A could be measured from the samples of the subjects who were given the emulgator-containing product. This result further supports the surprising observation that an emulsified oil product is not at all absorbed.

The test products contained no cholesterol. However, a clear difference between products A and A0 was observed in the cholesterol values. This result further supports the observations about the non-absorbability of emulsified oil, since fat absorption affects the organism's own cholesterol metabolism.

### EXAMPLE 3

### EFFECT OF EMULGATOR DOSE ON FAT ABSORPTION

In this experiment, the subjects ingested different amounts of emulgator-containing products. The test products were products A and B, prepared in Example 1, wherein the amount of E494 was 0.1% (A1 and B1), 0.4% (A2 and B2), 0.98% (A3 and B3) or 0% (A0 and B0). The experiment and the sampling were arranged as was described in Example 2.

The amount of test product ingested was proportional to the weights of the subjects such that the emulgator dose was according to Table 1. Thus, the average single dose was 490 ml.

**TABLE 1**

| Test product | g/skin-m² | g/weight-kg |
|---|---|---|
| A0 | 0 | 0 |
| A1 | 0.25 | 6.5 |
| A2 | 1.0 | 25 |
| A3 | 2.5 | 65 |
| B0 | 0 | 0 |
| B1 | 0.25 | 6.5 |
| B2 | 1.0 | 25 |
| B3 | 2.5 | 65 |

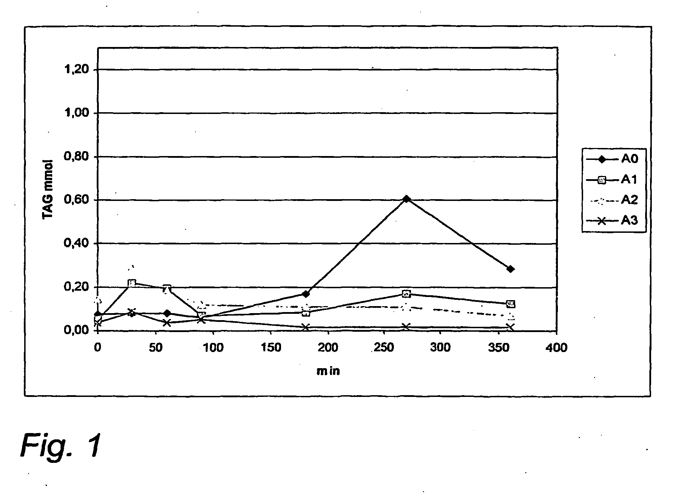

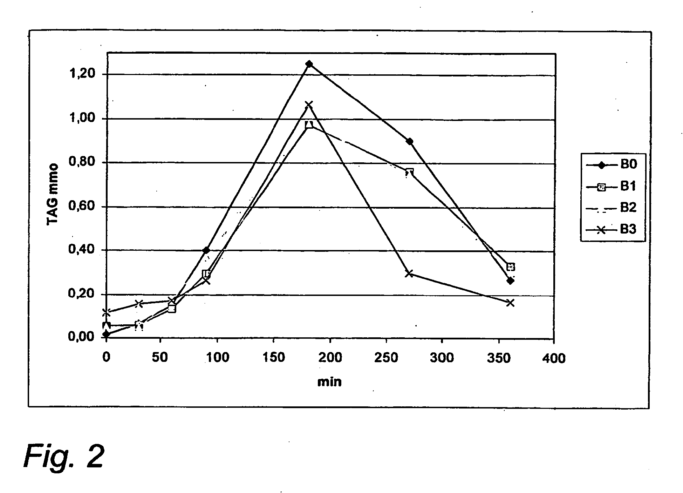

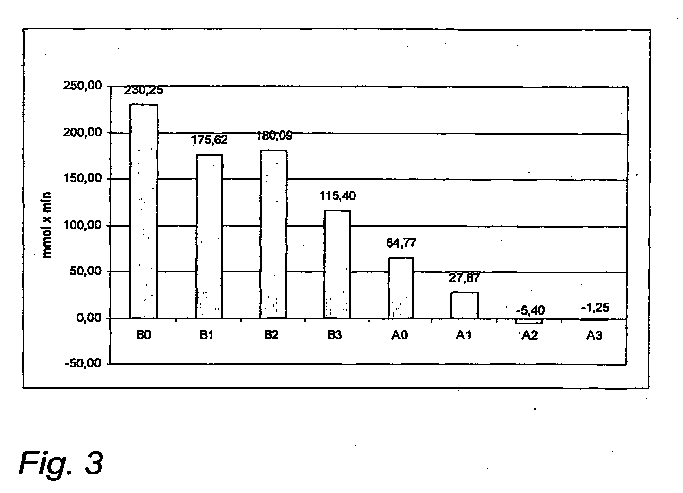

Figure 1 shows blood fat values during 0 to 6 hours for subjects that ingested test product A, and Figure 2 for subjects that ingested test product B. Figure 3 is a bar diagram showing the area (iAUC) remaining under the curves of Figures 1 and 2, which is an indicator of the amount of fat absorbed by a person and generally employed in the field. As the figures show, the dosage levels used in this example have a linear effect regarding product A. As regards product B, the effect is visible at all dosage levels, but on levels B1 and B2, the effect is equal. This refers to a possible plateau on the dose-response curve. The effect of the test products on the prevention of fat absorption was already clearly shown at lower dosage levels.

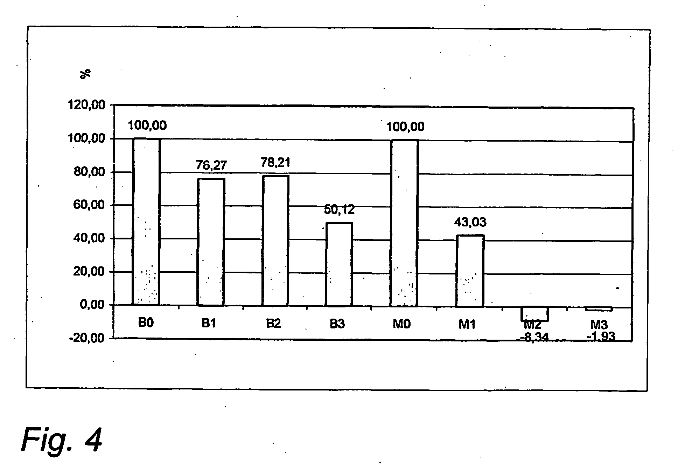

Figure 4 shows that the achieved prevention of fat absorption is proportional to the dose of the emulgator (100% = B0 and A0, which contained no emulgator at all).

As regards product B, the lower total level is likely to be due to the product being more complex (contains proteins and complex carbohydrates, for example), whereby the effect on fat absorption is not as strong as for product A. On the other hand, the effect manifested by product B shows that the functional food of the invention achieves the desired effect.

It is obvious to a person skilled in the art that as technology advances, the basic idea of the invention can be implemented in a variety of ways. The invention and its embodiments are thus not limited to the above examples, but may vary within the scope of the claims.

### REFERENCES

Zhi J; Melia A T; Guerciolini R; Chung J; Kinberg J; Hauptman J B; Patel I H Retrospective population-based analysis of the dose-response (fecal fat excretion) relationship of orlistat in normal and obese volunteers. Clinical Pharmacology And Therapeutics 1994, 56(1), 82-5.
NCCDPHP (National Center for Chronic Disease Prevention and Health Promotion), 29.8.2003 11:00 GMT.
   http://www.cdc.gov/nccdphp/dnpa/obesity/defining.htm
WHO, Technical Report Series 916. Diet, Nutrition and the Prevention of Chronic Diseases. Geneva 2003.
IASO 2003, http://www.iotf.org/media/iotfjan29.htm

## Claims

1. Food-approved sorbitane derivative for use as a pharmaceutical.

2. Use of a food-approved sorbitan derivative for the preparation of a therapeutic product intended for the prevention of fat absorption.

3. The use as claimed in claim 2, **characterized in that** the sorbitan derivative is sorbitan monooleate (E 494).

4. The use as claimed in claim 2, **characterized in that** the product is a pharmaceutical.

5. The use as claimed in claim 2, **characterized in that** the product is a food supplement.

6. The use as claimed in claim 2, **characterized in that** the product is a functional food.

7. The use according to any one of claims 2-6, **characterized in that** the product contains 1 to 10,000 mg, preferably 100 to 5,000 mg, more preferably 500 to 5,000 mg, and most preferably 2,000 mg of said sorbitan derivative per single dose ingested.

8. The use as claimed in any one of claims 2-7, **characterized in that** the product is intended for the prevention and treatment of obesity.

9. Use of a sorbitan derivative for the prevention of fat absorption for cosmetic weight control.

10. The use as claimed in claim 9, wherein said sorbitan derivative is sorbitan monooleate (E494).

## Patentansprüche

1. Für Nahrungsmittel zugelassenes Sorbitanderivat für die Verwendung als ein Pharmazeutikum.

2. Verwendung eines für Nahrungsmittel zugelassenen Sorbitanderivats für die Herstellung eines therapeutischen Produkts, welches zur Vorbeugung von Fettabsorption gedacht ist.

3. Verwendung wie in Anspruch 2 beansprucht, **dadurch gekennzeichnet, dass** das Sorbitanderivat Sorbitanmonooleat (E 494) ist.

4. Verwendung wie in Anspruch 2 beansprucht, **dadurch gekennzeichnet, dass** das Produkt ein Pharmazeutikum ist.

5. Verwendung wie in Anspruch 2 beansprucht, **dadurch gekennzeichnet, dass** das Produkt ein Nahrungsergänzungsmittel ist.

6. Verwendung wie in Anspruch 2 beansprucht, **dadurch gekennzeichnet, dass** das Produkt ein funktionelles Nahrungsmittel ist.

7. Verwendung gemäß irgendeinem der Ansprüche 2-6, **dadurch gekennzeichnet, dass** das Produkt 1 bis 10 000 mg, vorzugsweise 100 bis 5 000 mg, stärker bevorzugt 500 bis 5 000 mg und besonders bevorzugt 2 000 mg dieses Sorbitanderivats pro einzelner aufgenommener Dosis enthält.

8. Verwendung wie in irgendeinem der Ansprüche 2-7 beansprucht, **dadurch gekennzeichnet, dass** das Produkt für die Vorbeugung und Behandlung von Fettleibigkeit gedacht ist.

9. Verwendung eines Sorbitanderivats für die Vorbeugung von Fettabsorption zur kosmetischen Gewichtskontrolle.

10. Verwendung wie in Anspruch 9 beansprucht, wobei dieses Sorbitanderivat Sorbitanmonooleat (E 494) ist.

## Revendications

1. Dérivé de sorbitane alimentaire pour un usage en tant que produit pharmaceutique.

2. Utilisation d'un dérivé de sorbitane alimentaire pour la préparation d'un produit thérapeutique destiné à la prévention de l'absorption des graisses.

3. Utilisation selon la revendication 2, **caractérisée en ce que** le dérivé de sorbitane est le monooléate de sorbitane (E 494).

4. Utilisation selon la revendication 2, **caractérisée en ce que** le produit est un produit pharmaceutique.

5. Utilisation selon la revendication 2, **caractérisée en ce que** le produit est un complément alimentaire.

6. Utilisation selon la revendication 2, **caractérisée en ce que** le produit est un aliment fonctionnel.

7. Utilisation selon l'une quelconque des revendications 2 à 6, **caractérisé en ce que** le produit contient de 1 à 10 000 mg, de préférence de 100 à 5 000 mg, de manière encore préférée de 500 à 5 000 mg, et de manière la plus préférée 2 000 mg dudit dérivé de sorbitane par dose unique ingérée.

8. Utilisation selon l'une quelconque des revendications 2 à 7, **caractérisé en ce que** le produit est destiné à la prévention et au traitement de l'obésité.

9. Utilisation d'un dérivé de sorbitane pour la prévention de l'absorption de graisse en vue d'un contrôle du poids à des fins cosmétiques.

10. Utilisation selon la revendication 9, dans lequel ledit dérivé de sorbitane est le monooléate de sorbitane (E494).
